Europäisches Patentamt

European Patent Office

Office européen des brevets

(19)

(11) Publication number: **0 193 399**
**A2**

(12) **EUROPEAN PATENT APPLICATION**

(21) Application number: 86301390.0

(22) Date of filing: 26.02.86

(51) Int. Cl.⁴: **A 61 B 17/12**
**A 61 M 1/00**

(30) Priority: 26.02.85 JP 38563/85

(43) Date of publication of application:
03.09.86 Bulletin 86/36

(84) Designated Contracting States:
DE FR GB

(71) Applicant: KURARAY CO., LTD.
1621 Sakazu
Kurashiki-City Okayama Prefecture(JP)

(72) Inventor: Gion, Hidenori
638-3 Otogo
Okayama-city Okayama-Pref. 704(JP)

(72) Inventor. Yoda, Takumi
2-6-3 Fukushima
Okayama-city Okayama-Pref. 702(JP)

(72) Inventor: Kirita, Yasuzo
1-31 Kita-Midorigaoka
Toyonaka-city Osaka-Pref. 560(JP)

(72) Inventor: Zaima, Kazuhito
1241-99 Iioka
Saijo-city Ehime-Pref. 793(JP)

(72) Inventor: Kurata, Noriyuki
505-2 Maeda-Higashimachi
Takamatsu-city Kagawa-Pref. 760(JP)

(74) Representative: Topps, Ronald et al,
D. YOUNG & CO 10 Staple Inn
London WC1V 7RD(GB)

(54) **Apparatus for drawing blood from a limb.**

(57) An apparatus for drawing blood from an arm or leg is provided with an inflatable air bag (1) placed around the arm or leg and intermittently squeezes a part of the arm or leg, control of inflation and contraction of the air bag (1) being controlled by means of an electronic circuit (11) which permits free and precise change of the length of time for inflating or contracting the air bag as well as pressure in the air bag (1), the apparatus according to this invention for drawing blood from a swollen vein extending within a part of the limb between the air bag (1) and the extremity of the limb can increase the quantity of blood to be withdrawn. Therefore, this apparatus is suitable to be used together with an extracorporeal blood circulation and treating apparatus.

./...

# FIG. 1.

- 1
- air pump — 2
- external synchronous input circuit — 15
- signal input means — 10
- pressure sensor — 4
- 13
- (c)
- pump drive circuit — 9
- (a)
- 11
- pressure supply/air release cycle setting circuit
- (b)
- valve drive circuit — 12
- amplifier — 5
- 22 pressure supply/air release cycle control circuit
- (b)
- coincidence circuit — 8
- (g)
- (f)
- (d)
- (e)
- comparator circuit — 7
- pressure setting circuit — 6
- 20 control device
- 21 pressure control circuit

1

# APPARATUS FOR DRAWING BLOOD FROM A LIMB

This invention relates to an apparatus for drawing blood from a patient's arm or leg. The apparatus of this invention can increase a quantity of blood drawn per unit time and, therefore, is particularly suitable for the joint use with a blood treatment apparatus for plasma collection requiring a long period of time in blood drawing.

A method generally employed for drawing blood from the patient's body makes use of a tourniquet in the form of a rubber tube. The tourniquet is tightly wound around the patient's arm or leg. Blood is drawn from a swollen vein within the part of the limb between the tourniquet and the extremity of the arm or leg. In most cases, blood is drawn from the region of the end of an elbow. In the case of extracorporeal circulation blood treatment, treated blood is returned to the patient's body through either the other arm which is free of a tourniquet or a part lying upstream of the tourniquet.

Such a method as using the tourniquet is well known to those skilled in the art, however, there is a problem in that the method is not suitable for extracorporeal blood circulation continuing for a long time due to a possible danger of toxic substances staying in the vein pressed by the tourniquet. Accordingly, when using the tourniquet, it is necessary to loosen the tourniquet periodically and then re-start extracorporeal blood circulation. However, in practice, adjustment of pressure for tightening the tourniquet during blood drawing is difficult.

On the other hand, a method of pressing the vein with a bag-like band, instead of the tourniquet, wound around the patient's arm or leg and inflated with air is now employed (Japanese Patent Laid-Open No. 12195/1983 and others). According to this method, the bag-like band is connected by a tube to a container containing pressurising gas such as nitrogen or Freon and the pressurising gas is fed into the bag-like band by opening an electrically operated valve connected to the tube. This method, however, is attended by a danger of toxic substances staying in the pressed part of the vein due to constant inflation of the band similarly to the application of the tourniquet. In addition, the use of a pressurising gas is

2

0193399

dangerous and has the problem that a sure and safe adjustment of pressure requires the use of a large and costly apparatus.

The apparatus of this invention intermittently presses the patient's vein for increasing a quantity of blood to be drawn while inflating and contracting a bag-like band wound around the patient's arm or leg.

According to the present invention there is provided an apparatus for drawing blood from a limb of a patient, in which air is fed from a pneumatic pump into a bag-like band placed around the patient's limb so as to squeeze a part of the arm or leg and a valve is opened for air discharge from the air bag at the same time with the stoppage of the pump, characterised in that the apparatus includes a control circuit which provides signals for periodically closing said valve in response to external signals and at the same time emitting signals for driving the pump and controls the interval of pressurising of and air discharge from the air bag; a pressure control circuit which compares the pressure of the air bag with a preset level of pressure and emits signals for driving the pump until the air bag pressure reaches the preset level; and a coincidence circuit which emits pump driving signals to the pump driving circuit only when signals emitted by the control circuit for controlling the interval of pressurising and air discharge from the air bag and signals emitted by the circuit for controlling the air bag pressure are all common signals such as signals for driving the pump.

An embodiment of the invention will now be described, by way of an example, with reference to the accompanying drawings, in which:-

Figure 1 is a schematic block diagram of an embodiment of an apparatus according to the present invention;

Figure 2 is a time chart representing the performance of parts of the apparatus;

Figure 3 is an electrical circuit of the pressurising/air-discharge interval setting circuit shown in Figure 1;

Figure 4 is a perspective view of an apparatus of this invention connected to the patient's body;

Figure 5 is a graph showing the relation between a length of time for pressurising the cuff and the quantity of let blood;

Figure 6 is a graph showing the relation between the pressure for pressurising the cuff and the quantity of let blood;

Figure 7 is a flow chart showing an example using an apparatus of this invention together with a plasma collection apparatus; and

Figure 8 is a flow chart showing an example using an apparatus of this invention together with a haemodialysis apparatus.

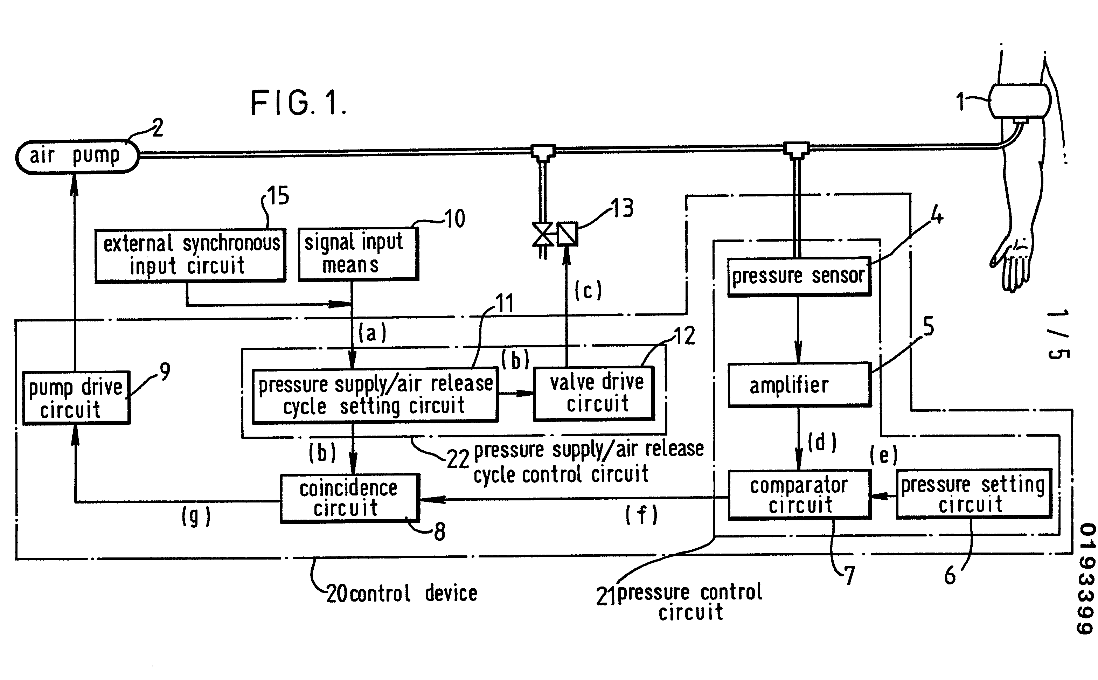

Figure 1 is a schematic block diagram of an apparatus according to this invention.

The main components of the apparatus comprise a bag-like band 1 extending around and pressing against the patient's arm or leg and which applies and releases pressure to and from the patient's vein while periodically repeating inflation and contraction thereof; a pneumatic pump 2 actuated only when inflating the bag-like band 1, and feeding air into the bag-like band 1 through a conduit 14; a valve 13 for feeding air into the air bag or discharging air therefrom while discharging or keeping pressurised air generated by driving of the abovesaid pump 2; and a control device 20 for emitting opening/closure signals to the abovesaid valve 13 and drive/stop signals to the pneumatic pump 2.

The bag-like band 1 is preferably an inflatable air bag which can be wound around the patient's arm or leg and capable of squeezing a part of the arm or leg over a fixed length, which is generally a cuff of the kind used for blood pressure measurement used with a sphygmomanometer. The width of the cuff for blood pressure measurement is usually 14 cm for adults.

When pricking one arm with two needles for drawing and returning of blood, particularly, in the case of haemodialysis using shunts, it is preferable to apply the cuff to a part of the arm between the blood drawing needle and blood returning one for easier blood returning. In this case, a cuff of small width, usually 4 to 9 cm, is used.

The pneumatic pump 2 may be any of those which are capable of obtaining a flow quantity of 2 l/min or more and pressure of 100 mmHg or more, such as a diaphragm pump and bellows pump of small size. The pump 2 is not always driven throughout the use of this apparatus but, when required, driven through a control device that will be described later. Air fed from the pump 2 passes through the conduit 14 and inflates the cuff 1.

The valve 13 connected to the conduit 14 is opened and closed by electric or pneumatic signals and usually comprises an electromagnetic valve. The valve 13 is opened upon stoppage of the pneumatic pump 2 and discharges air from the cuff 1 for contraction thereof. This valve 13 is of a type which is opened in usual time and closed when electrically powered.

The control device 20 for emitting opening/closure signals to the valve 13 and drive/stop signals to the pneumatic pump 2 is composed of a pressure control circuit 21 for controlling the cuff pressure and a pressurising/discharge interval control circuit 22 for periodically opening and closing the valve 13.

The pressure control circuit 21 for controlling the cuff pressure is composed of a pressure sensor 4 (usually, a semiconductor pressure transducer and a differential transformer type pressure transducer) which senses the air pressure in the conduit 14 and converts pressure energy into electric signals; an amplifier 5 for amplifying the signals produced by the abovesaid sensor 4; a pressure setting circuit 6 for setting the cuff pressure; and a comparator circuit 7 which compares the amplified signals transmitted from the pressure sensor 4 with the pressure signals set by the pressure setting circuit 6 and emits pump driving signals until the cuff pressure reaches a set level of pressure and pump stopping signals when the cuff pressure reaches the set level.

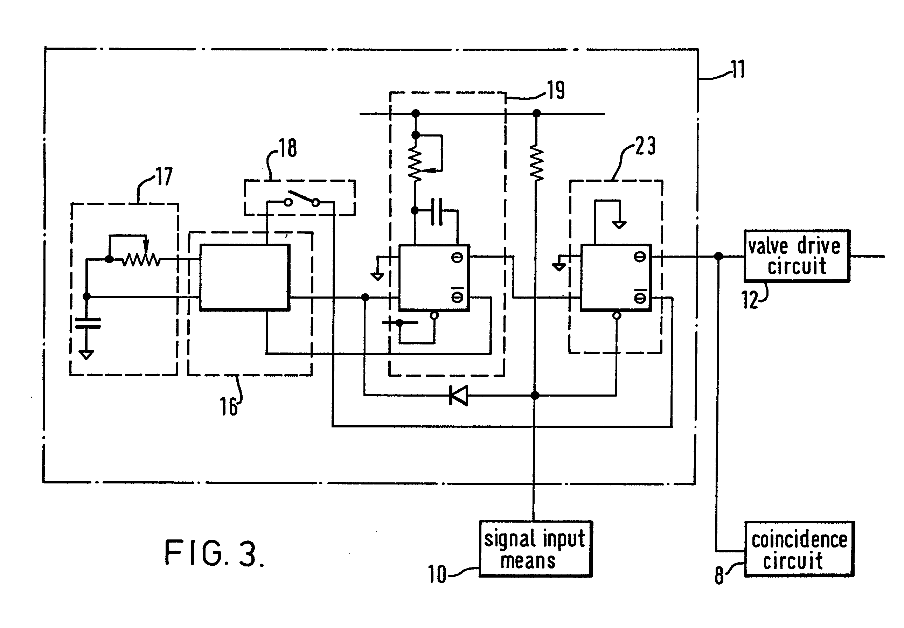

On the other hand, the pressurising/discharge interval control circuit 22 sets periods of time inflating (pressurising) the cuff 1 and releasing air pressure (contraction) for opening and closing the valve 13 with the valve opening/closing signals emitted therefrom. The circuit 22 comprises a circuit 11 for setting a time for inflating and contracting the cuff 1 and a valve driving circuit 12 for opening and closing the valve 13 in response to signals from the circuit 11. An example of a circuit 11 for setting a time of cuff inflation and contraction is shown in Figure 3. The circuit of Figure 3 has an oscillator and divider circuit 16 which generates a square wave according to the cuff pressing interval which is adjusted by an interval time set circuit 17. A mode switch 18, which is the part of the interval time set circuit 17, decides the cuff pressing mode (continuous pressing/intermittent pressing). The pressing/releasing time control circuit 11 decides the cuff pressing time and the cuff releasing time (fixed by a releasing time set circuit 19).

A logic circuit 23 emits ON/OFF signals to a coincidence circuit 8 and the valve driving circuit 12.

Pump driving signals are emitted from the pressurising/discharge interval setting circuit 11 to the valve driving circuit 12 and further to the coincidence circuit 8 as shown in Figure 1. These pump driving signals are compared with those emitted from the pressure control circuit 21 and, only when they are pump driving signals together, pump driving signals are emitted to the pump driving circuit 9, thereby the pump 2 being driven. When at least one of these two kinds of signals is for pump stopping (cuff contraction), no signals are emitted from the coincidence circuit 8 to the pump driving circuit 9, thereby the pump 2 is stopped.

For any of the abovesaid circuits, various types of known circuits may be used.

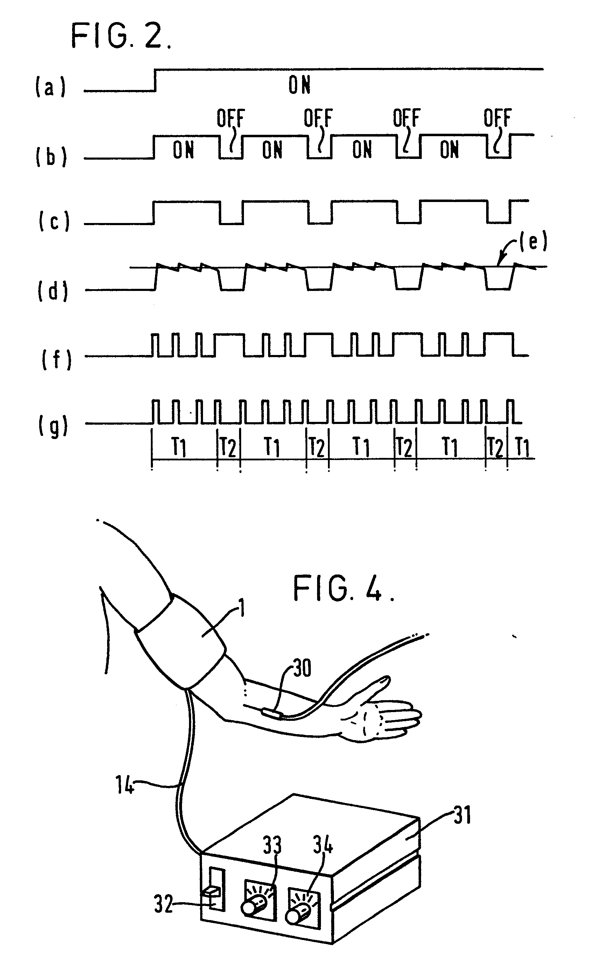

Operation of the apparatus of this invention will be described with reference to a time chart shown in Figure 2. At first, ON signals (a) are emitted from signal input means, for example, an ON/OFF button 10, to the abovesaid pressurising/discharge interval control circuit 22. When synchronising ON signals with external signals, it is necessary to further provide an external synchronous input circuit 15. If so provided, valve opening/closing signals (b) are periodically emitted from the pressurising/ discharge interval setting circuit 11 for setting a period of time $T_1$ for inflation and time $T_2$ for contraction to the valve driving circuit 12. The valve driving signals (b) are emitted toward the coincidence circuit 8, too, and compared with signals (c) emitted from the pressure control circuit 21 at the coincidence circuit 8.

On the other hand, the cuff pressure emits pump driving signals (f) until signals (d), which are emitted from the pressure sensor 4, amplified by the amplifier 5, and compared with a preset pressure level (e) by the comparator circuit 7, reaches this pressure level (e). The cuff used in the sphygmomanometer is made of cloth and attended by air leakage which also occurs in the pneumatic pump and, therefore, pump driving signals are intermittently emitted for constantly maintaining the cuff pressure at a preset level during the cuff inflation time $T_1$. Signals (f) from the pressure control circuit 21 and those (b) from the pressurising/discharge interval setting circuit 11 are compared with each other by the coincidence circuit 8 and, only when these two kinds of signals are all pump driving signals, pump driving signals (g) are emitted to the pump driving circuit 9 for driving the pump 2. When at least one of the two kinds of signals is not the pump driving one (pump stoppage), no signals are transmitted from the coincidence circuit to the pump driving circuit. Accordingly, during the time as above, the pump 2 is not driven, that is, the cuff contracts. This

6

0193399

apparatus detects abnormal pressure, if any, with a pressure sensor and, while emitting alarm in response to detection signals, stops operating so as to discharge air from the cuff. Control parts of the pressurising/discharge interval setting circuit 11 and the coincidence circuit 8 may jointly be controlled by means of a microcomputer and adapted to operate according to a predetermined program.

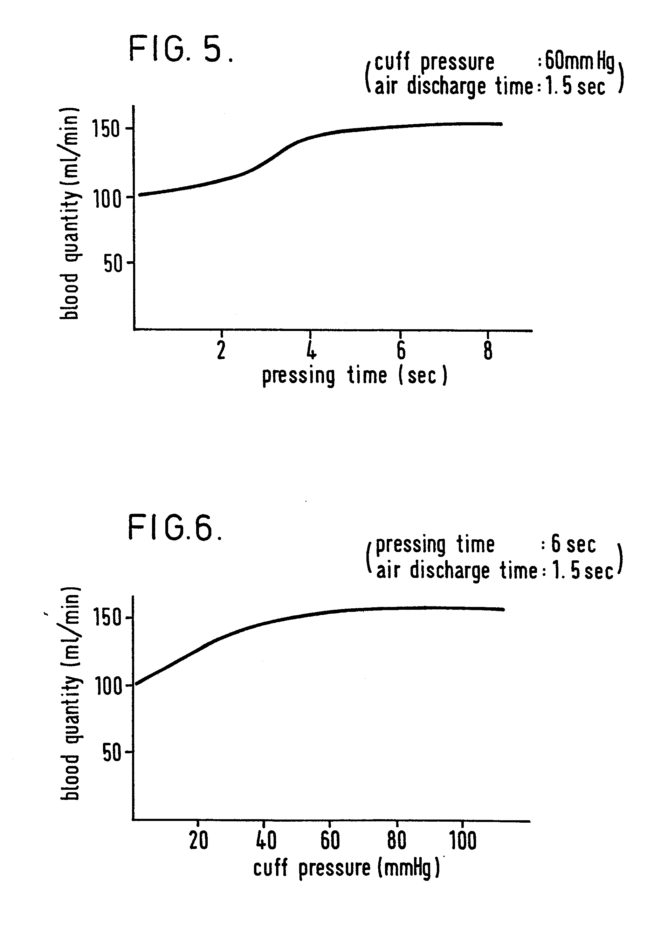

The above-mentioned apparatus can easily be connected to the patient. Figure 4 shows an example of the apparatus connected to the patient, wherein the cuff 1 is fitted on an upper arm of the patient. An injection needle or catheter 30 for returning blood to the patient's body is arranged near the elbow. The reference numeral 31 indicates a monitor unit containing the pneumatic pump 2, valve 13, and control device 20 as shown in Figure 1, the monitor 31 being connected to the cuff 1 through a conduit 14 preferably made of neoprene rubber. The monitor unit used in this invention is, for example, 15 cm wide, 8 cm high, and 16 cm long, and provided with a power switch 32 disposed on the front side, a dial 33 for setting the pressing time within the range from 0 to 10 sec and a pressure setting dial 34 for setting a cuff pressure within the range from 0 to 150 mmHg. Further, in this apparatus, the air discharge time is set at 1.5 sec in the monitor unit beforehand and this can be changed by the operator from outside the unit. The air distance time is usually set within the range from 1 to 3 sec. A length of time shorter than 1 sec leads to a possibility that pressing is performed earlier than complete discharge of air from the cuff 1 whereas a length of time longer than 3 sec results in a low rate of increase in the quantity of blood to be drawn per unit time.

An apparatus of this invention is used at a cuff pressure ranging from 30 to 150 mmHg and the pressing time ranging from 3 to 10 sec by the use of a cuff. Such conditions as above has been decided as will be described hereinunder. A result of examining the relation between the length of pressing time and the drawn blood quantity at a cuff pressure of 60 mmHg and the air discharge time of 1.5 sec are shown in Figure 5. As apparent from Figure 5, there is no significant increase in the quantity of drawn blood with a pressing time shorter than 3 sec. With a pressing time longer than 10 sec, the blood quantity is in equilibrium and does not increase further. A result of examining the relation between the cuff pressure and the quantity of drawn blood under the abovesaid conditions is shown in Figure 6. As apparent from Figure 6, there is no significant increase in blood quantity at a cuff pressure lower than 30 mmHg. When the cuff pressure was 150 mmHg or higher, the patient felt a strong pressing force and, in addition, no increase in the quantity of drawn blood was achieved. From the above results, it has been found that, when setting the cuff pressure at 30 to 150 mmHg and the pressing time of the cuff at 3 to 10 sec, the use of this apparatus improves the flow quantity of blood in a vein to 70 to 80 ml/min as compared with 40 to 50 ml/min at the time of blood drawing without the use of any tourniquet.

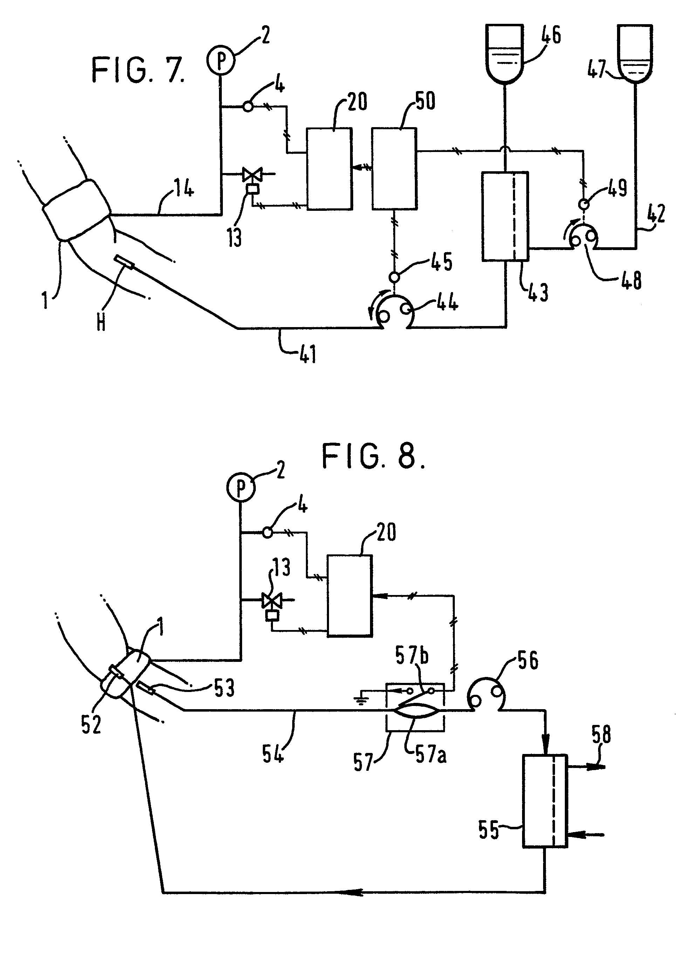

This apparatus is not limited to the use for blood treatment and can be used, as an example, for plasma separation to separate plasma component from blood components (red blood corpuscle, while blood corpuscle and blood platelet) using centrifugal force or membrane. In the case of plasma removal operation, plasma component is drawn from the patient. Further, plasma removal operation for therapeutic purpose can be referred to as an example. In this case, the plasma component is separated from blood components of the patient. At the time of such plasma removal operation, the plasma component is treated for removal of certain toxic substance in plasma of the patient and then continuously returned to the body of the same patient together with blood components. The apparatus of this invention can be used for haemodialysis using a comparatively small flow quantity of blood. The apparatus of this invention is controlled in association with a blood treatment apparatus and constantly operated at the time of blood drawing. At this time, an external synchronising circuit is provided and external signals are input thereinto. For example, when the apparatus is used for collecting plasma component from blood according to a membrane separation method, signals are supplied to the control device 20 only at the time of drawing blood. In response to the signals, the cuff 1 fitted on the upper arm of the patient intermittently presses against the vein of the patient. In the apparatus shown in Figure 7, a blood flow passage 41 is provided with a membrane module 43 for separating blood discharged from the blood drawing needle H into corpuscle and plasma components, a bag 46 for receiving the corpuscular component separated by the membrane module 43, a reversible blood pump 44, and a sensor 45 for sensing the flow quantity in the blood passage, whereby the separated corpuscular component is fed into the bag 46 and the blood pump 44 is driven in the reverse

0193399

direction for returning corpuscular component in the bag 46 to the patient's body through the injection needle H.

The plasma flow passage 42 is provided with a bag 47 for receiving the plasma component separated by the membrane module 43 and a sensor 49 for sensing the flow quantity in the plasma flow passage, whereby plasma component separated by the membrane module 43 is reserved in the bag 47.

The blood pump 44 stops drawing blood when receiving sensing signals from the blood flow quantity sensor 45 and rotates in the reverse direction for returning corpuscular component in the corpuscle reserving bag 46 to the patient's body. When revolutions of the blood pump 44 in the reverse direction in a fixed length of time have been detected by the timer or the like, the blood pump 44 again rotates in the forward direction for drawing blood from the patient. With the repetition of the abovesaid performance, a required quantity of plasma component is collected. The blood pump 44 is driven by means of the control device 50. The apparatus of this invention is of such design that signals are supplied from the abovesaid control device 50 into a control device 20 when the blood pump 44 rotates in the right direction.

In the process of extracorporeal blood treatment, it is also possible to intermittently inflate the cuff by emitting signals when the quantity of blood drawn from the patient decreases and by inputting these signals into the control device 20. In this case, signals emitted to the control device 20 diminishes with the return of the quantity of drawn blood to the normal flow quantity. Decrease in quantity of drawn blood can be detected by, for example, a pressure sensor, usually a bag-like pillow switch, disposed upstream of the blood pump in the blood circulation flow passage and detecting the negative pressure in the flow passage. The pillow switch is always fixed to the extracorporeal blood circulation passage and an example of application thereof will be described with reference to blood dialysis treatment shown in Figure 8. In the case of haemodialysis, usual shunts are used and accordingly the cuff 1 is attached to a part between the shunt 52 on the artery side and that 53 on the vein side. Therefore, a cuff 1 of narrow width as ranging from 3 to 5 cm is used so as to permit easy fitting on the part between the two shunts 52, 53. The blood flow passage 54 is provided with a membrane module 55 for dialysing blood drawing through the shunt 53 on the vein side by a blood pump 56, and returns purified blood

**0193399**

to the patient's body through the shunt 52 on the artery side. The numeral 58 indicates a flow passage for dialysate. A pillow switch 57 serves as a detector for detecting the negative pressure in the flow passage is disposed upstream of the pump 56. The pillow switch 57 comprises a pillow sensor 57a consisting of an inflatable/contractable bag-like band and a contact 57b which is in contact with the pillow sensor 57a and opened and closed with inflation and contraction, respectively, of the pillow sensor 57a. When the quantity of drawn blood decreases during therapy, a negative pressure is generated by suctional force of the blood pump 56 in the flow passage between the shunt 53 on the vein side and the blood pump 56. The bag-like pillow sensor 57a of the pillow switch 57 contracts, as shown in the drawing, due to the abovesaid negative pressure, whereby the contact 57b is opened and negative pressure sensing signals are generated. In response to such sensing signals, the control circuit 20 acts for intermittently inflating the cuff 1. With intermittently pressing exerted on the patient's vein and the increase in quantity of drawn blood, the pillow sensor 57a returns to the initial state while inflating, and closes the contact 57b, thereby negative pressure sensing signals diminishing. In this way, the control device 20 becomes out of operation and the cuff 1 contracts.

As hitherto described, an apparatus of this invention is suitable for extracorporeal blood treatment of all kinds. The apparatus, when the air discharge time is set at zero, permits continuous pressing and, therefore, provides optimum method for the patient's conditions in such a way as continuous pressing at the beginning of blood drawing and subsequent intermittent pressing.

## CLAIMS

1.      An apparatus for drawing blood from a limb of a patient, in which air is fed from a pneumatic pump (2) into a bag-like band (1) placed around the patient's limb so as to squeeze a part of the arm or leg and a valve (13) is opened for air discharge from the air bag (1) at the same time with the stoppage of the pump (2), characterised in that the apparatus includes a control circuit (22) which provides signals for periodically closing said valve (13) in response to external signals and at the same time emitting signals for driving the pump (2) and controls the interval of pressurising of and air discharge from the air bag (1); a pressure control circuit (21) which compares the pressure of the air bag (1) with a preset level of pressure and emits signals for driving the pump (2) until the air bag pressure reaches the preset level; and a coincidence circuit (8) which emits pump driving signals to the pump driving circuit (9) only when signals emitted by the control circuit (22) for controlling the interval of pressurising and air discharge from the air bag (1) and signals emitted by the circuit (21) for controlling the air bag pressure are all common signals such as signals for driving the pump (2).

2.      An apparatus as claimed in Claim 1, further characterised in that the control circuit (22) for controlling the interval of pressurising of and air discharge from the air bag (1) is provided with an external synchronous input circuit (15).

3.      An apparatus as claimed in Claim 2, in which the external synchronous input circuit (15) receives blood drawing signals transmitted ⁻ from the blood treatment apparatus and inflates the air bag (1) during the process of blood drawing.

4.      An apparatus as claimed in Claim 2, in which the external synchronous input circuit (15), when receiving negative pressure sensing signals from the pressure sensor sensing the negative pressure generated in the blood passage of the blood treatment apparatus, inflates the air bag (1).

## FIG. 1.

pressure supply/air release cycle control circuit

pressure control circuit

control device

# FIG. 2.

(a) ON

(b) ON OFF ON OFF ON OFF ON OFF

(c)

(d) (e)

(f)

(g) T1 T2 T1 T2 T1 T2 T1 T2 T1

# FIG. 4.

1

30

14

31

33 34

32

FIG. 3.

signal input means

10

valve drive circuit

12

coincidence circuit

8

3/5

0193399

# FIG. 5.

$\left(\begin{array}{l}\text{cuff pressure} \quad : 60\,\text{mm Hg} \\ \text{air discharge time} : 1.5\,\text{sec}\end{array}\right)$

blood quantity (mL/min)

150

100

50

2  4  6  8

pressing time (sec)

# FIG.6.

$\left(\begin{array}{l}\text{pressing time} \quad : 6\,\text{sec} \\ \text{air discharge time} : 1.5\,\text{sec}\end{array}\right)$

blood quantity (mL/min)

150

100

50

20  40  60  80  100

cuff pressure (mmHg)

FIG. 7.

FIG. 8.